# EUROPÄISCHE PATENTANMELDUNG

(11) **EP 2 380 560 A1**
(43) Veröffentlichungstag der Anmeldung: **26.10.2011**
(21) Anmeldenummer: 10004284.5
(22) Anmeldetag: 22.04.2010
(51) Int. Cl.: A61K 9/20, A61K 9/00, A61K 31/425

(54) **Pramipexol enthaltende Matrixtablette**

(71) Anmelder: ratiopharm GmbH, 89079 Ulm (DE)
(72) Erfinder: Rimkus, Katrin, D-80798 München (DE); Stumm, Daniela, 83730 Fischbachau (DE)
(74) Vertreter: TER MEER - STEINMEISTER & PARTNER GbR

(57) **Zusammenfassung**

Die vorliegende Erfindung betrifft eine Matrixtablette zur modifizierten Freisetzung von Pramipexol, sowie ein Verfahren zur Herstellung solcher Matrixtabletten. Die Erfindung betrifft darüber hinaus Pramipexol enthaltende Matrixtabletten zur Behandlung von Morbus Parkinson.

## Beschreibung

Die vorliegende Erfindung betrifft eine Matrixtablette zur modifizierten Freisetzung von Pramipexol, sowie ein Verfahren zur Herstellung solcher Matrixtabletten. Die Erfindung betrifft darüber hinaus Pramipexol enthaltende Matrixtabletten zur Behandlung von Morbus Parkinson.

*(S*)-2-Amino-6-(propylamino)-4,5,6,7-tetrahydrobenzothiazol ist unter dem INN-Namen "Pramipexol" bekannt und weist folgende Strukturformel auf:

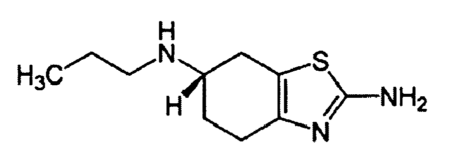

Pramipexol ist ein Dopaminagonist, der derzeit bereits zur symptomatischen Behandlung des idiopathischen Morbus Parkinson eingesetzt wird. Bei Morbus Parkinson handelt es sich um eine progressive Erkrankung des Gehirns, die sich durch Zittern, langsame Bewegungen und Muskelsteife äußert. Bei Patienten, die an Morbus Parkinson erkrankt sind, beginnen Dopamin produzierende Zellen abzusterben, so dass die Menge an Dopamin im Gehirn abnimmt. Dopamin ist als Botenstoff in der Steuerung von Bewegung und Koordination involviert. Ein Dopaminmangel hat zur Folge, dass die Patienten die Fähigkeit zur Steuerung verlieren. Pramipexol ist als Dopaminagonist ein Stoff, der die Wirkung von Dopamin nachahmt und das Gehirn genauso oder ähnlich stimuliert wie Dopamin, so dass die Symptome von Morbus Parkinson gelindert werden.

Zusätzlich zur Behandlung von Morbus Parkinson kann Pramipexol eingesetzt werden zur Behandlung des sogenannten Restless-Legs-Syndroms, des Syndroms der ruhelosen Beine. Dabei handelt es sich um eine Erkrankung, bei der die Patienten, gewöhnlich nachts, den unwiderstehlichen Drang haben, die Glieder zu bewegen, um sich eines als unangenehm bis schmerzhaft empfundenen Gefühls in den Gliedern zu entledigen.

Pramipexol enthaltende Tabletten sind in Deutschland derzeit bereits unter dem Handelsnamen Sifrol^{®} erhältlich. Neben Tabletten zur sofortigen Freisetzung sind unter diesem Handelsnamen auch Retardtabletten erhältlich, und zwar in den Wirkstärken 0,26 mg, 0,52 mg, 1,05 mg, 2,1 mg und 3,15 mg Pramipexol. Die Retardtabletten enthalten als pharmazeutische Hilfsstoffe Hypromellose (Hydroxypropylmethylcellulose HPMC), Maisstärke, Carbomer 941 (Polyacrylsäure), hochdisperses Siliciumdioxid und Magnesiumstearat. Die retardierende Wirkung beruht dabei auf der Verwendung zweier Polymere: Carbomer 941 als einem nicht-quellenden, pH-abhängigen Polymer und Hypromellose als einem quellenden, pH-unabhängigen Polymer. Entsprechende Formulierungen werden beispielsweise in den internationalen Anmeldungen WO 2007/090881 und WO 2006/0159942 vorgeschlagen.

Diese Retardtabletten weisen eine ganze Reihe von Nachteilen auf. Zum Einen müssen die Tabletten eine gewisse Mindestgröße aufweisen, um die notwendige Quellung des quellenden Polymers zu ermöglichen. Dies ist nötig, um eine gewünschte Diffusion gemäß dem ersten Fick'schen Gesetz zu erreichen. Des Weiteren macht sich eine starke Nahrungs- und Flüssigkeitsabhängigkeit bemerkbar, da für eine ausreichende Quellung der Tablette im Körper eine ausreichende Flüssigkeitsmenge zur Verfügung stehen muss. Ferner neigen Polymere in hohen Mengen zur Bildung toxischer Abbauprodukte und zum Nachsintern, was das Freisetzungsverhalten in unvorteilhafter Weise beeinflusst.

Es ist daher eine Aufgabe der vorliegenden Erfindung, einen oder mehrere der oben genannten Nachteile zu überwinden oder zumindest zu mindern. Es soll insbesondere ein Retardtablette bereitgestellt werden, die Pramipexol im Wesentlichen unabhängig von Nahrungs- und Flüssigkeitsaufnahme freisetzt.

Diese Aufgabe konnte gelöst werden durch die erfindungsgemäße Matrixtablette und ein Verfahren zu deren Herstellung.

Gegenstand der vorliegenden Erfindung ist eine Matrixtablette zur modifizierten Freisetzung von Pramipexol, umfassend:
a) 0,05 bis 5 Gew.-% Pramipexol oder ein pharmazeutisch akzeptables Salz davon,
b) 40 bis 80 Gew.-% eines Matrixbildners, der ausgewählt ist aus der Gruppe bestehend aus Wachsen, Fetten, Ölen, Fettsäuren mit mehr als 12 Kohlenstoffatomen, Fettalkoholen, Monoglyceriden, Diglyceriden, Triglyceriden und Gemischen davon,
c) 10 bis 50 Gew.-% Tablettiermittel,
d) 0 bis 25 Gew.-% Matrixmodifikator,
e) 0 bis 5 Gew.-% Fließregulierungsmittel, und
f) 0 bis 5 Gew.-% Schmiermittel,
bezogen auf das Gesamtgewicht der Matrixtablette.

Das Gesamtgewicht der Matrixtablette bezieht sich hierin auf die Matrixtablette an sich, das heißt eine unbefilmte bzw. unbelackte Matrixtablette. Sofern nichts anderes angegeben ist, beziehen sich die prozentualen Gewichtsangaben hierin auf das Gesamtgewicht der Matrixtablette.

Gegenstand der vorliegenden Erfindung ist ferner ein Verfahren zum Herstellen einer erfindungsgemäßen Matrixtablette zur modifizierten Freisetzung von Pramipexol, umfassend
(i) Herstellen eines Gemischs umfassend Pramipexol oder ein pharmazeutisch akzeptables Salz davon a), Matrixbildner b), Tablettiermittel c), gegebenenfalls Matrixmodifikator d), gegebenenfalls Fließregulierungsmittel e) und gegebenenfalls Schmiermittel f),
(ii) Kompression des in Schritt (i) erhaltenen Gemischs.

Ein weiterer Gegenstand der Erfindung ist eine erfindungsgemäße Matrixtablette zur Behandlung von Morbus Parkinson.

Die Modifizierung der Freisetzung bzw. die Retardwirkung der erfindungsgemäßen Matrixtabletten beruht alleinig oder zumindest im Wesentlichen auf einer Einbettung von Pramipexol in eine Matrix aus den oben genannten Matrixbildnern, nämlich insbesondere Fetten und Wachsen und diesen verwandten und ähnlichen Stoffen. Diese besonderen Matrixbildner haben den Vorteil, dass sie im Vergleich zu den im Stand der Technik, insbesondere in Sifrol^{®}, verwendeten Polymeren im Körper besser abgebaut werden können, da der Abbau enzymatisch über Lipasen erfolgt und somit unabhängig von der vorhandenen Wassermenge im Gastrointestinaltrakt immer gleichmäßig stattfinden kann. Neben den Matrixbildnern können optional vergleichsweise geringe Mengen an Matrixmodifikatoren in der Matrix vorhanden sein, mit deren Hilfe sich die Eigenschaften der Matrix und damit die Kinetik der Freisetzung des Wirkstoffs aus der Matrix einstellen lassen.

Erfindungsgemäß können 0,05 bis 5 Gew.-% Pramipexol oder ein pharmazeutisch akzeptables Salz davon in der Matrixtablette enthalten sein. Dabei kann es sich um ein Salz oder auch ein Gemisch von Salzen handeln. Bevorzugt werden als Salze Säureadditionssalze verstanden, wie beispielsweise Hydrochloride, Carbonate, Hydrogencarbonate, Acetate, Lactate, Butyrate, Propionate, Sulfate, Methansulfonate, Citrate, Tartrate, Nitrate, Sulfonate, Oxalate und/oder Succinate. Ferner sollen vom Begriff Pramipexol und dessen Salzen auch pharmazeutisch akzeptable Solvate, insbesondere Hydrate, umfasst sein. Besonders bevorzugt wird Pramipexoldihydrochlorid-Monohydrat eingesetzt. Pramipexol kann in amorpher oder kristalliner Form in der Matrixtablette enthalten sein.

Der Einfachheit halber wird in der folgenden Beschreibung "Pramipexol" stellvertretend sowohl für die freie Base als auch für Salze und Solvate davon verwendet. Bevorzugt ist Pramipexol in einer Menge von weniger als 1 Gew.% in der Matrixtablette enthalten, bevorzugt bis zu maximal einschließlich 0,94 Gew.-% oder maximal einschließlich 0,92 Gew.-%.

Weiter bevorzugt sind Matrixtabletten mit einer Menge an Pramipexol, bezogen auf die freie Base, von 0,26 mg, 0,52 mg, 1,05 mg, 2,1 mg oder 3,15 mg. Die Menge von 3,15 mg freier Base von Pramipexol entspricht dabei einer Menge von 4,5 mg Pramipexoldihydrochlorid-Monohydrat, um nur ein Beispiel zu nennen.

Pramipexol kann beispielsweise in einer Form eingesetzt werden, in der 90 Volumen-% der Pramipexolpartikel eine kleinere Partikelgröße aufweisen als 190 µm (D₉₀ = 190 µm) und/oder 50 Volumen-% der Pramipexolpartikel eine kleinere Partikelgröße aufweisen als 80 µm (D₅₀ = 80 µm) und/oder 10 Volumen-% der Pramipexolpartikel eine kleinere Partikelgröße aufweisen als 21 µm (D₁₀ = 21 µm). "Partikelgröße" bezeichnet hierin den Durchmesser eines äquivalenten Partikels, von dem man annimmt, dass es kugelförmig ist und dass es das gleiche Lichtstreuungsmuster aufweist wie das zu bestimmende Partikel. Die Partikelgröße wird hierin mit Laserdiffraktometrie bestimmt, beispielsweise mit Hilfe eines Mastersizer 2000 von Malvern Instruments. Bevorzugt ist eine Nassmessung an einer Dispersion von Partikeln (2000 UpM, Ultraschall 60 Sekunden, Abschattung von 4 bis 15 %). Die Auswertung erfolgt generell für Partikel mit einem D₅₀- Wert von kleiner als 5,0 µm mit Hilfe der Mie Methode und für Partikel mit einem D₅₀-Wert von mindestens 5,0 µm mit Hilfe der Fraunhofer Methode.

Pramipexol kann in einer weiteren beispielhaften Ausführungsform in mikronisierter Form vorliegen, wobei beispielsweise 90 Volumen-% der Pramipexolpartikel eine kleinere Partikelgröße aufweisen als 30 µm (D₉₀) oder beispielsweise kleiner als 20 µm (D₉₀).

Matrixbildner sind gemäß der vorliegenden Erfindung Wachs, Fett, Öl, Fettsäure, Fettalkohol, Monoglycerid, Diglycerid, Triglycerid und Gemische davon. Der Matrixbildner kann einen oder mehrere dieser Stoffe enthalten. Die Definitionen der genannten Matrixbildner überlappen zu einem gewissen Maß und sind nachfolgend aufgeführt:

Triglyceride sind Ester des dreiwertigen Alkohols Glycerol (Glycerin, Propan-1,2,3-triol) mit drei Carbonsäureresten (Triester, auch bezeichnet als Acylglycerine). Die Carbonsäurereste können gleich oder verschieden sein. Diglyceride sind Ester von Glycerol mit zwei Carbonsäureresten, d.h. es sind nur zwei der drei Hydroxy-Gruppen des Glycerols verestert (Diester). Je nach Stellung der Carbonsäurereste wird zwischen 1,2- und 1,3-Diglyeriden unterschieden. Auch hier können die Carbonsäurereste gleich oder verschieden sein. Monoglyceride sind Ester von Glycerol mit nur einem Carbonsäurerest, d.h. es ist nur eine der drei Hydroxy-Gruppen des Glycerols verestert (Monoester).

Beispiele für erfindungsgemäß einsetzbare Monoglyceride sind etwa: Glycerin(mono)behenat (2,3-Dihydroxypropyldocosanat), Glycerinmonostearat, Glycerinmonocaprat, Glycernmonococoat, Glycerinmonoerucat, Glycerinmonohydroxystearat, Glycerinmonoisosterat, Glycerinmonolanolat, Glycerinmonolaurat, Glycerinmonolinoleat, Glycerinmonomyristat, Glycerinmonooleat, Glycerinmonopalmitat, Glycerinmonoricinoleat, Glycerin(mono)myristat, Glycerin(mono)montanat und Gemische davon, wie etwa Glycerinpalmitatstearat, dem Monoester des Glycerins mit einem Gemisch aus Palmitin- und Stearinsäure.

Beispiele für erfindungsgemäß einsetzbare Diglyceride sind etwa Diester mit den gleichen Carbonsäureresten wie die für die Monoglyceride genannten, zum Beispiel Glycerindilaurat, Glycerindimyristat, Glycerindioleat, Glycerindipalmitat, Glycerindistearat, Glycerindiisostearat, und Gemische davon und Diester mit unterschiedlichen Carbonsäureresten (gemischte Diester) wie etwa Glycerinpalmitostearat (Precirol^{®}) und Gemische davon.

Erfindungsgemäß einsetzbare Triglyceride umfassen beispielsweise Glycerintricaprylsäureester, Glycerintrilaurat, Glycerintrioleat, Glycerintriricinoleat, Glycerintristearat und Gemische davon.

Erfindungsgemäß können auch Gemische von Mono- und Diglyceriden verwendet werden, wie etwa Glycerinmono- und -dioleat oder Glycerinmono- und -distearat. Ferner können Gemische von Mono- und Triglyceriden, Gemische von Di- und Triglyceriden oder Gemische von Mono-, Di- und Triglyceriden eingesetzt werden. Es können jedoch auch Mono-, Di- und Triglyceride jeweils allein eingesetzt werden, das heißt Triglyceride in Abwesenheit von Mono- und/oder Diglyceriden, Diglyceride in Abwesenheit von Mono- und/oder Triglyceriden und Monoglyceride in Abwesenheit von Di- und/oder Triglyceriden.

Bevorzugte Beispiele von Glyceriden bzw. Glyceridgemischen sind Glycerinmonobehenat (Compritol^{®}), Glycerinpalmitostearat (Precirol^{®}), Glycerinmonostearat (Cutina GMS^{®}), Neutralöl (Gemisch aus kurz- und mittelkettigen Triglyceriden, vorwiegend mit den Fettsäuren Capryl- und Caprinsäure; Miglyol^{®}) und Gemische davon.

Unter "Fetten" versteht man Ester von Glycerol (Glycerin) mit drei Fettsäuren, also einer bestimmten Gruppe von Carbonsäuren. Es handelt sich also um eine Untergruppe der Triglyceride. Die Fettsäuren in dem Ester eines Fetts können verschieden (gemischte Glyceride) oder aber auch gleich sein. Die Fettsäuren können gesättigt oder ungesättigt sein, und geradzahlig oder ungeradzahlig sein. Die physikalischen Eigenschaften eines Fettes werden durch die Kettenlängen und insbesondere durch die Häufigkeit von Doppelbindungen, d.h. den Grad der Sättigung, in den Fettsäuren bestimmt. Fette können bei Raumtemperatur und Normaldruck (25°C, 1013 hPa) fest oder flüssig sein. Fette, die bei Raumtemperatur und Normaldruck (25°C, 1013 hPa) flüssig sind, bezeichnet man auch als fette Öle. Die tierischen Fette enthalten hauptsächlich gemischte Glyceride von drei Säuren, nämlich von Palmitin-, Stearin- und Ölsäure. Die pflanzlichen Öle enthalten außer Glyceriden der Palmitin-, Stearin- und Ölsäure vor allem Glycerinester der mehrfach ungesättigten Säuren. Beispiele für pflanzliche Öle sind Sesamöl, Olivenöl, Mandelöl, Maisöl, Palmöl, Erdnußöl, Kokosöl, Rapsöl, Weizenkeimöl, Hanföl, Mohnöl, Leinöl, Rizinusöl, Sonnenblumenöl, Baumwollsamenöl, und Sojabohnenöl. Es sind vorliegend auch hydrierte bzw. gehärtete Fette und Öle umfasst, beispielsweise gehärtetes bzw. hydriertes Rizinusöl (z.B. Cutina HR^{®}), welches eine bevorzugte Ausführungsform der vorliegenden Matrixbildner darstellt. Weniger bevorzugt bzw. vorteilhaft nicht enthalten sind derivatisierte Fette wie beispielsweise polyoxyethylierte Fette.

Fettsäuren sind gesättigte oder ungesättigte Carbonsäuren. Die erfindungsgemäß einsetzbaren Fettsäuren mit mehr als 12 Kohlenstoffatomen werden als höhere Fettsäuren bezeichnet. Fettsäuren sind in der Regel und hierin bevorzugt unverzweigt, d.h. geradkettig. Beispiele für erfindungsgemäß verwendbare Fettsäuren sind: Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure, Palmitoleinsäure, Ölsäure, Erucasäure, Linolsäure, Linolensäure, Elaeostearinsäure, Arachidonsäure und Clupanodonsäure. Eine bevorzugte Fettsäure ist Stearinsäure.

Öle umfassen im Rahmen der vorliegenden Erfindung die vorstehend bereits genannten fetten Öle, flüssiges Paraffin sowie flüssige Propylenglycoldiester von Fettsäuren, wie z.B. Miglyol^{®} 840.

Fettalkohole sind lineare, gesättigte oder ungesättigte primäre Alkohole (1-Alkanole) mit mindestens sechs Kohlenstoffatomen, bevorzugt 6 bis 22 Kohlenstoffatomen. Sie werden meist durch Reduktion aus Fettsäuren gewonnen. Hierin werden den Fettalkoholen auch solche zugerechnet, die mehr als 22 Kohlenstoffatome aufweisen und zuweilen als "Wachsalkohole" bezeichnet werden. Beispiele für Fettalkohole sind: Capronalkohol (1-Hexanol), Önanthalkohol (1-Heptanol), Caprylalkohol (1-Octanol), Pelargonalkohol (1-Nonanol), Caprinalkohol (1-Decanol), 1-Undecanol, 10-Undecen-1-ol, Laurylalkohol (1-Dodecanol), 1-Tridecanol. Myristylalkohol (1-Tetradecanol, 1-Pentadecanol, Cetylalkohol (1-Hexadecanol), 1-Heptadecanol, Stearylalkohol (1-Octadecanol), Oleylalkohol (9-*cis*-Octadecen-1-ol), Erucylalkohol (9-*trans-*Octadecen-1-ol), Ricinolalkohol (9-*cis*-Octadecen-1,12-diol), Linoleylalkohol *(all-cis-9,12-* Octadecadien-1-ol), Linolenylalkohol (*all-cis*-9,12,15-Octadecatrien-1-ol), 1-Nonadecanol, Arachidylalkohol (1-Eicosanol), Gadoleylalkohol (9-*cis*-Eicosen-1-ol), 5,8,1,14-Eicosentetraen-1-ol, 1-Heneicosnol, Behenylalkohol (1-Docosanol), Erucylalkohol (1-3*cis*-Docosen-1-ol), Brassidylalkohol (1-3*trans*-Docosen-1-ol), Lignocerylalkohol, Cerylalkohol, Myricylalkohol. Ein bevorzugtes Beispiel ist Stearylalkohol.

Wachse werden typischerweise phänomenologisch als solche Substanzen verstanden, die bei 20°C (und 1013 hPa) knetbar, fest bis brüchig hart sind, eine grobe bis feinkristalline Struktur aufweisen, durchscheinend bis opak, jedoch nicht glasartig sind, über 40°C (und 1013 hPa) ohne Zersetzung schmelzen und schon wenig oberhalb ihres Schmelzpunktes niedrigviskos sind. Sie weisen eine stark temperaturabhängige Konsistenz und Löslichkeit auf und sind unter leichtem Druck polierbar. Wachse sind hierein bevorzugt ferner ausgewählt aus der Gruppe bestehend aus oder umfassend Ester von Fettsäuren, bevorzugt höheren Fettsäuren (C>12), mit Alkoholen mit Ausnahme von Glycerin, bevorzugt langkettigen, aliphatischen Alkoholen, insbesondere den oben genannten Wachsalkoholen (C>22), oder Gemischen aus solchen Estern. Wachse können bei Raumtemperatur und Normaldruck (25°C, 1013 hPa) fest oder flüssig sein. Beispiele für Wachse sind etwa natürliche Wachse wie pflanzliche, tierische oder petrochemische Wachse. Beispiele dafür sind etwa Carnaubawachs, Myrtenwachs, Japanwachs, Zuckerrohrwachs (pflanzlich), Bienenwachs (tierisch), Paraffinwachs oder mikrokristallines Wachs (petrochemische Wachse). Bevorzugt nicht umfasst vom Begriff Wachs sind vorliegend sogenannte synthetische Wachse.

Erfindungsgemäß können auch Lösungen von Wachsen in organischen Ölen oder dünnflüssiges Wachs verwendet werden.

Bevorzugt werden solche Matrixbildner b) eingesetzt, die bei Raumtemperatur und Normaldruck (25°C, 1013 hPa) fest sind.

In bevorzugten Ausführungsformen ist der Matrixbildner b) ausgewählt aus der Gruppe bestehend aus Carnaubawachs, Stearinsäure, Glycerolbehenat, Glycerolmonostearat, gehärtetem Rizinusöl und Gemischen davon. Bevorzugt wird beispielsweise Glycerinbehenat in Kombination mit Stearinsäure eingesetzt, oder Carnaubawachs in Kombination mit Stearinsäure, oder eine Kombination von Glycerolbehenat, hydriertem Rizinusöl und Glycerinmonostearat. In vorteilhaften Ausführungsformen kann auch Stearinsäure als alleiniger Matrixbildner eingesetzt werden. Im Allgemeinen erlaubt eine Kombination mehrerer Matrixbildner eine fein einstellbare Steuerung der Freisetzungseigenschaften der Matrix, während die Verwendung eines einzigen Matrixbildners den Vorteil eines vereinfachten Herstellungsverfahrens hat.

Es werden mindestens 40 Gew.-%, bezogen auf das Gesamtgewicht der Matrixtablette, der genannten Matrixbildner b) eingesetzt, bevorzugt mehr als 40 Gew.-%, beispielsweise mindestens 40,5 Gew.-%, etwa mindestens 41 Gew.-% und bevorzugt mehr als 45 Gew.%, beispielsweise mindestens 45,5 Gew.-%. In vorteilhaften Ausführungsformen kann der Gehalt an Matrixbildner b) bei mehr als 50 Gew.%, beispielsweise mindestens 50,5 Gew.-%, bevorzugt mehr als 55 Gew.% und besonders bevorzugt mehr als 60 Gew.-% betragen. Dabei werden maximal 80 Gew.-% Matrixbildner b) eingesetzt, bevorzugt bis einschließlich 75, 74, 70 oder 69 Gew.-%.

In Ausführungsformen, in denen Stearinsäure als Matrixbildner b) oder Teil des Matrixbildners b) verwendet wird, macht der Matrixbildner bevorzugt zwischen 45 und 65 Gew.% des Gesamtgewichts der Matrixtablette aus. Wird Stearinsäure beispielsweise mit einem anderen Matrixbildner, wie zum Beispiel Glycerinbehenat oder Carnaubawachs, als Matrixbildner b) eingesetzt, so liegt die verwendete Menge an Stearinsäure bevorzugt bei 25 bis 35 Gew.-% und die Gesamtmenge an Matrixbildner b) bei 50 bis 70 Gew.-%, besonders bevorzugt 55 bis 65 Gew.-%, jeweils bezogen auf das Gesamtgewicht der Matrixtablette.

Glycerinbehenat wird bevorzugt in Kombination mit mindestens einem weiteren Matrixbildner b) eingesetzt. Eine Kombination aus Glycerinbehenat und einem einzigen weiteren Matrixbildner macht bevorzugt mindestens 55 Gew.-% des Gesamtgewichts der Matrixtablette aus, wobei auf das Glycerinbehenat bevorzugt ein Anteil von mindestens 20 bis 40 Gew.-% des Gesamtgewichts der Matrixtablette, d.h. etwa die Hälfte des Gewichts der Matrixbildner oder mehr entfällt. Eine Kombination aus Glycerinbehenat und zwei weiteren Matrixbildnern macht bevorzugt mindestens 55 Gew.-% des Gesamtgewichts der Matrixtablette aus, wobei auf das Glycerinbehenat bevorzugt ein Anteil von 15 bis 35 Gew.-%, bevorzugt 17,5 bis 25,0 Gew.-% des Gesamtgewichts der Matrixtablette entfällt.

Wird eine Kombination von zwei Matrixbildnern b) eingesetzt, so erfolgt dies beispielsweise in einem Verhältnis der Gewichtsanteile des ersten und zweiten Matrixbildners von 10 : 1 bis 1 : 10, beispielsweise 2 : 1 bis 1 : 2, bevorzugt 1,5 : 1 bis 1 : 1,5, und noch stärker bevorzugt 1,25 : 1 bis 1: 1,25. Dies gilt bevorzugt insbesondere für Kombinationen eines Matrixbildners b) mit Glycerinbehenat, Carnaubawachs oder Stearinsäure, beispielweise die Kombinationen von Glycerinbehenat und Carnaubawachs oder die Kombination von Glycerinbehenat und Stearinsäure.

Wird eine Kombination von drei Matrixbildnern b) eingesetzt, so erfolgt dies beispielsweise in einem Verhältnis der Gewichtsanteile bzw. Gewichtsanteilsbereiche des ersten zu zweiten zu dritten Matrixbildners von 1 : (0,1 bis 10) : (0,1 bis 10), bevorzugt 1 : (0,5 bis 2) : (0,5 bis 2), und stärker bevorzugt 1: (0,75 bis 1,25) : (0,75 bis 1,25).

Optional kann zusätzlich zu dem Matrixbildner b) ein Matrixmodifikator d) eingesetzt werden, und zwar bis einschließlich 25 Gew.-% des Gesamtgewichts der Matrixtablette. In beispielhaften Ausführungsformen ist der Matrixmodifikator d) in Mengen von 1 bis 22 Gew.-%, bevorzugt 3 bis einschließlich 20 Gew.-% des Gesamtgewichts der Matrixtablette enthalten.

Wird ein Matrixmodifikator d) eingesetzt, so umfasst oder ist der Matrixmodifikator d) bevorzugt ein pH-abhängiger Hilfsstoff, bevorzugt ein pH-abhängiges Polymer oder ein Gemisch pH-abhängiger Hilfsstoffe oder bevorzugt pH-abhängiger Polymere. Bevorzugt besteht der Matrixmodifikator d) aus einem einzigen pH-abhängigen Polymer.

Unter einem pH-abhängigen Hilfsstoff, wie zum Beispiel einem Polymer, werden im Rahmen der vorliegenden Erfindung solche Hilfsstoffe verstanden, deren retardierende Wirkung bei verschiedenen pH-Werten unterschiedlich ist, d.h. die bei verschiedenen pH-Werten (z.B. pH 1,2 und pH 6,8) den Wirkstoff unterschiedlich schnell freisetzen. Bevorzugt wird der pH-abhängige Hilfsstoff so ausgewählt, dass die Freisetzung von Pramipexol aus der Matrixtablette in den ersten 4 Stunden bei pH 1 bis pH 6,8 unter Verwendung der USP Testapparatur I (Körbchen) und einer Drehgeschwindigkeit von 100 Umdrehungen pro Minute in 750 - 1000 ml Medium bei einer Temperatur von 37°C auf höchstens 50 % verringert wird.

Der Matrixmodifikator d) kann beispielsweise ausgewählt sein aus der folgenden Gruppe pH-abhängiger Hilfsstoffe:
- Polymeren der Acrylsäure (Polyacrylsäure) und deren Derivate, Polyacrylaten und deren Derivaten, Copolymeren aus Methacrylsäure und Methacrylsäuremethylester,
- hydrophilen Polymeren auf Cellulosebasis, beispielsweise Celluloseestern und Celluloseethern sowie deren Salzen und Derivaten, z.B. Carboxymethylcellulose-Natrium, Carboxymethylcellulose-Calcium, Carboxymethylethylcellulose-Natrium, Carboxymethylethylcellulose-Calcium, Celluloseacetatphthalat, Cellulosediacetatphthalat, Cellulosetriacetatphthalat, Hydroxypropylmethylcellulosephthalat, Natriumcelluloseacetatphthalat, Celluloseesterphthalat, Celluloseetherphthalat, Celluloseesteretherphthalat, Hydroxypropylcellulosephthalat, Methylcellulosephthalat,
- Polysacchariden, wie z.B. Alginaten, Gummi arabicum, Xanthan Gummi, Chitinderivaten wie Chitosan, und Carrageenan,
- Keratin,
- und Gemischen davon.

Bevorzugte Matrixmodifikatoren d) sind Polysaccharide und Polymere der Acrylsäure (Polyacrylsäure), wobei Carrageenan besonders bevorzugt ist. Carrageenan ist ein Polysaccharid-Auszug aus Carrageen (getrockneten, nordatlantischen Rotalgen). Es handelt sich bei Carrageenan um einen anionischen Polyelektrolyten mit hoher elektrischer Ladung. Bevorzugte Polymere der Acrylsäure sind unter der Handelsbezeichnung Carbopol^{®} oder Carbomer bekannt. Geeignet sind beispielsweise Polyacrylsäuren mit einer Viskosität einer 0,5% (w/v) Lösung in Wasser von 4 000 - 40 000 mPa•s.

Als besonders vorteilhaft haben sich eine Kombination von Polyacrylsäure (Carbopol^{®}), Stearinsäure und Glycerinbehenat (Compritol^{®}) und eine Kombination von Polyacrylsäure (Carbopol^{®}), Carnaubawachs und Stearinsäure erwiesen.

Werden hydrophile Polymere aus der Gruppe Polyacrylsäure oder deren Derivate, Polyacrylate oder deren Derivate, oder Copolymere aus Methacrylsäure und Methacrylsäureester, bevorzugt jeweils allein oder in Kombination, als Matrixmodifikator d) eingesetzt, so machen diese Polymere bevorzugt 1,0 bis 10,0 Gew.%, mehr bevorzugt 2,0 bis 7,5 Gew.%, und am meisten bevorzugt 2,5 bis 5,0 Gew.%, des Gesamtgewichts der Matrixtablette aus. Werden diese hydrophilen Polymere als alleinige Matrixmodifikatoren d) eingesetzt, so werden sie vorteilhaft in einem Gewichtsverhältnis von Matrixmodifikator d) zu Matrixbildner b) eingesetzt, der im Bereich von beispielsweise 1 : 10 bis 1 : 30, bevorzugt 1 : 12 bis 1 : 25 und besonders bevorzugt zwischen 1 : 15 bis 1 : 18 liegt.

Wird ein Polysaccharid oder Polysaccharidgemisch, wie z.B. Carrageenan, bevorzugt allein, als Matrixmodifikator d) eingesetzt, so macht es bevorzugt 5,0 bis 25,0 Gew.%, mehr bevorzugt 7,5 bis 22,5 Gew.%, und am meisten bevorzugt 9,0 bis 21,0 Gew.%, des Gesamtgewichts der Matrixtablette aus.

Als besonders vorteilhaft hat sich eine Kombination aus Carrageenan und Stearinsäure herausgestellt. In solchen Kombinationen macht der Anteil von Carrageenan am Gesamtgewicht der Matrixtablette bevorzugt 2,0 bis 5,0 Gew.-% aus, der Anteil von Stearinsäure bevorzugt 45,0 bis 65,0 Gew.-%.

Der Matrixmodifikator d) wirkt in bevorzugten Ausführungsformen als Dochtmittel. Im Allgemeinen versteht man unter einem Dochtmittel ein Mittel mit der Fähigkeit, eine biologische Flüssigkeit (bevorzugt Wasser) in einen Feststoff zu ziehen, beispielsweise mittels Physisorption. Physisorption wird definiert als Form der Adsorption, bei welcher die Flüssigkeitsmoleküle an die Oberfläche des Dochtmittels anhaften können, bevorzugt mittels van-der-Waals-Bindung zwischen der Oberfläche des Dochtmittels und dem adsorbierten flüssigen Molekül (bevorzugt Wasser). Ein Dochtmittel kann dies mit oder ohne Aufquellen bewirken. Ein Dochtmittel kann in den erfindungsgemäßen Matrixtabletten die Bildung von Kanälen oder Poren in der Matrix bzw. der Matrixtablette bewirken bzw. fördern. Dies kann das Eindringen der Wassermoleküle erleichtern, insbesondere durch Physisorption. Daher besteht die Funktion des Dochtmittels darin, Wasser ins Innere der Matrixtablette zu transportieren, um dadurch Kanäle in oder ein Netzwerk auf einer vergrößerten Oberfläche zu schaffen. Durch die Verwendung eines Matrixmodifikators und insbesondere Dochtmittels können die Zerfallseigenschaften der Matrix insgesamt und dadurch das Freisetzungsverhalten der Matrix bzw. der Matrixtablette in vorteilhafter Weise gesteuert werden.

Die erfindungsgemäße Matrixtablette enthält ferner ein Tablettiermittel c) in einer Menge von 10 bis 50 Gew.-%, bevorzugt 15 bis 45 Gew.% und beispielsweise 17,5 bis 42,5 Gew.-%.

Das Tablettiermittel c) ist bevorzugt ausgewählt aus den im Fachbereich üblichen pharmazeutischen Füllstoffen. Dies sind typischerweise Stoffe, die zur Bildung des Körpers der oralen Darreichungsform bei Darreichungsformen mit kleinen Wirkstoffmengen erforderlich sind, um eine ausreichende Menge an Darreichungsmasse für eine geeignete Darreichungsgröße zu erhalten.

Als Tablettiermittel c) können beispielsweise Alkali- oder Erdalkalisalze wie Calciumphosphate, z.B. Calciumhydrogenphosphat (CaHPO₄, z.B. in Form des Dihydrats oder bevorzugt des Anhydrats), Calciumcarbonat, Magnesiumcarbonat, Magnesiumoxid, Calciumsulfat, Natriumchlorid, Kaliumchlorid, Lactose, Lactosederivate, Stärke, Stärkederivate, behandelte Stärke, Chitin, Cellulose und Derivate davon, z.B. mikrokristalline Cellulose (z.B. Avicel^{®}), Saccharose, Dextrate, Dextrin, Dextrose, Maltodextrin, Kaolin und Gemische davon verwendet werden. Ebenfalls kann SiO₂ modifizierte mikrokristalline Cellulose (z.B. Prosolv^{®}, Rettenmaier & Söhne, Deutschland) verwendet werden. Auch Zuckeralkohole und/oder Zucker (insbesondere Mono- und Disaccharide) wie Mannitol, Sorbitol, Xylitol, Isomalt, Glucose, Fructose, Maltose und Gemische daraus verwendet werden. Grundsätzlich können auch Gemische der genannten Tablettiermittel verwendet werden.

Bevorzugt wird als Tablettiermittel, bevorzugt als alleiniges Tablettiermittel, Calciumhydrogenphosphat (CaHPO₄ z.B. in Form des Anhydrats) eingesetzt.

Optional kann die erfindungsgemäße Matrixtablette auch ein Fließregulierungsmittel e) enthalten, bevorzugt bis zu einer Menge von 5 Gew.-% des Gesamtgewichts der Matrixtablette.

Fließregulierungsmittel haben allgemein die Aufgabe, in einem Tablettiergemisch sowohl die interpartikuläre Reibung (Kohäsion) zwischen den einzelnen Partikeln als auch das Haften dieser an den Wandflächen der Pressform (Adhäsion) zu vermindern. Ein Beispiel für ein Fließregulierungsmittel, also einen Zusatz zur Verbesserung der Pulverfließfähigkeit, ist disperses Siliciumdioxid (z.B. Aerosil^{®}). Bevorzugt wird Siliciumdioxid mit einer spezifischen Oberfläche von 50 bis 400 m²/g, bestimmt nach Gasadsorption gemäß Ph. Eur., 6. Auflage 2.9.26., verwendet. Das Fließregulierungsmittel e) kann aus einem Stoff oder aber einem Stoffgemisch bestehen.

Ein Fließregulierungsmittel e) kann beispielsweise in einer Menge von 0,1 bis 5 Gew.-%, bevorzugt 0,3 bis 3 Gew.-%, stärker bevorzugt 0,5 bis 2,5 Gew.-%, bezogen auf das Gesamtgewicht der Matrixtablette, verwendet werden.

Die Matrixtablette kann ferner ein Schmiermittel f) enthalten, bevorzugt in einer Menge bis zu 5 Gew.-%, bezogen auf das Gesamtgewicht der Matrixtablette. In bevorzugten Ausführungsformen enthält die Matrixtablette Schmiermittel f). Schmiermittel dienen im Allgemeinen zur Verringerung der Gleitreibung. Insbesondere soll die Gleitreibung vermindert werden, die beim Tablettieren einerseits zwischen den sich in der Matrizenbohrung auf- und ab bewegenden Stempeln und der Matrizenwand sowie andererseits zwischen Tablettensteg und Matrizenwand besteht. Geeignete Schmiermittel f) sind z.B. Natriumstearylfumarat (Pruv^{®}), Magnesiumstearat und/oder Calciumstearat. Magnesiumstearat ist dabei bevorzugt.

Das Schmiermittel f) kann beispielsweise in einer Menge von 0,1 bis 4 Gew.-%, bevorzugt 0,3 bis 3 Gew.-%, stärker bevorzugt 0,5 bis 2 Gew.-%, bezogen auf das Gesamtgewicht der Matrixtablette, verwendet werden.

Optional können weitere Hilfsstoffe in der erfindungsgemäßen Matrixtablette enthalten sein. In bevorzugten Ausführungsformen bestehen die Matrixtabletten jedoch aus den Bestandteilen a) bis f) in den angegebenen prozentualen Anteilen, ohne dass weitere Hilfsstoffe enthalten wären. Bevorzugt sind die Bestandteile a) bis f) voneinander verschieden, das heißt kein Inhaltsstoff erfüllt mehr als eine Funktion. Wird Stearinsäure beispielsweise als Matrixbildner eingesetzt, wird Stearinsäure bevorzugt nicht zusätzlich auch als Schmiermittel verwendet. Bevorzugt wird für jeden der Bestandteile c) bis f) der Matrixtablette jeweils nur ein Stoff, bevorzugt jeweils nur eine Verbindung eingesetzt.

Die Matrixtabletten sind dazu vorgesehen, den Wirkstoff modifiziert freizusetzen. In bevorzugten Ausführungsformen weist das Freisetzungsprofil der erfindungsgemäßen Matrixtabletten bei Messung unter Verwendung von Apparatur I (Körbchen) gemäß USP in Phosphatpuffer bei pH 6,8 (900 mL, 100 Umdrehungen pro Minute, 37°C) nach 1 Stunde einen freigesetzten Gehalt von mindestens etwa 10 % des Wirkstoffs und/oder nach 4 Stunden mindestens etwa 25 % des Wirkstoffs und/oder nach 8 Stunden mindestens etwa 40 % des Wirkstoffs und/oder nach 16 Stunden mindestens etwa 60 % des Wirkstoffs und/oder nach 24 Stunden mindestens etwa 80 % des Wirkstoffs auf. Ferner liegt das Freisetzungsprofil (gemäß obiger USP-Methode) bevorzugt in den nachstehend angegebenen Bereichen nach den einzelnen Zeiten, bevorzugt sind die Freisetzungsangaben für alle angegebenen Zeiten erfüllt:

| Zeit [h] | % freigesetzter Wirkstoff |
|---|---|
| 1 | 10-20 |
| 4 | 25-50 |
| 8 | 40-75 |
| 16 | ≥ 60 |
| 24 | ≥ 80 |

Ferner sind die erfindungsgemäßen Matrixtabletten besonders bevorzugt monolithisch, das heißt, sie bestehen aus einer einzigen diskreten Funktionseinheit. Dies ist im Gegensatz zu den sogenannten multipartikulären Systemen, bei denen eine Tablette in viele Untereinheiten zerfällt, die jeweils unabhängig voneinander den Wirkstoff freisetzen. Insbesondere sind keine sogenannten Aufbaupellets vorgesehen, bei denen der Wirkstoff auf inerte Starterkerne aufgebracht ist. Das heißt ferner, dass die Matrixtablette über ihren gesamten Querschnitt eine im Wesentlichen homogene Wirkstoff und Hilfsstoffverteilung aufweist. Insofern existiert auch nur ein Freisetzungsprofil für den Wirkstoff, es sind bevorzugt keine voneinander verschiedenen Retardierungsprinzipien oder -strukturen vorgesehen, die unterschiedliche Freisetzungsprofile für verschiedene Teile des Wirkstoffs bereitstellen würden.

Ausführungsformen der erfindungsgemäßen Matrixtabletten weisen bevorzugt eine Masse von 150 bis 600 mg, bevorzugt 200 bis 550 mg, oder besonders bevorzugt 240 bis 500 mg auf.

Im Rahmen der Erfindung können die resultierenden Tabletten beschichtet oder unbeschichtet (befilmt oder unbefilmt) vorliegen. Als Filmbildner für die Beschichtung können beispielsweise Cellulosederivate wie etwa Methylcellulose (MC), Ethylcellulose (EC), Hydroxyethylcellulose (HEC), Methacrylsäure-Acrylat-Copolymere wie beispielsweise Methacrylsäure-Ethacrylat-Copolymer oder Methacrylsäure-Methylmethacrylat-Copolymer, Vinylpolymere wie beispielsweise Polyvinylpyrrolidon oder Polyvinylacetatphthalat oder natürliche Filmbildner, wie beispielsweise Schellack, verwendet werden. Die Beschichtung enthält besonders bevorzugt kein Pramipexol. Die Dicke der Schicht, soweit vorhanden, beträgt üblicherweise 0,1 bis 100 µm, bevorzugt 1 bis 80 µm.

Es ist stark bevorzugt, dass die gegebenenfalls aufgebrachte Schicht im Wesentlichen keine Auswirkungen auf die Freisetzung hat. Somit handelt es sich bevorzugt um Schichten bzw. Filme ohne oder mit vernachlässigbarem Einfluss auf die Wirkstofffreisetzung. Im Rahmen dieser Erfindung werden bevorzugt weder magensaftresistente Filmüberzüge noch Retardüberzüge verwendet.

Die resultierenden Tabletten weisen bevorzugt eine Härte von 20 bis 150 N, besonders bevorzugt von 20 bis 100 N, insbesondere 25 bis 50 N auf. Die Härte wird gemäß Ph.Eur. 6.0, Abschnitt 2.9.8 bestimmt.

Des Weiteren weisen die resultierenden Tabletten bevorzugt eine niedrige Friabilität auf, nämlich beispielsweise eine Friabilität von 0,1 bis 0,8 %, bevorzugt 0,2 bis 0,6 % und besonders bevorzugt 0,3 bis 0,5 %. Die Friabilität wird gemäß Ph.Eur. 6.0, Abschnitt 2.9.7 bestimmt.

Wie bereits erwähnt, umfasst das erfindungsgemäße Verfahren zum Herstellen einer erfindungsgemäßen Matrixtablette zur modifizierten Freisetzung von Pramipexol folgende Schritte:
(i) Herstellen eines Gemischs umfassend Pramipexol oder ein pharmazeutisch akzeptables Salz davon a), Matrixbildner b), Tablettiermittel c), gegebenenfalls Matrixmodifikator d), gegebenenfalls Fließregulierungsmittel e) und gegebenenfalls Schmiermittel f),
(ii) Kompression des in Schritt (i) erhaltenen Gemischs zu einer Matrixtablette.

In Schritt (i) können optional weitere pharmazeutisch akzeptable Hilfsstoffe zugefügt werden, es ist jedoch in Analogie zu den obigen Ausführungen zur Matrixtablette bevorzugt, dass neben den Bestandteilen a) bis f) keine weiteren Hilfsstoffe eingesetzt werden. Im Übrigen gelten die für die erfindungsgemäßen Matrixtabletten gemachten Ausführungen entsprechend auch für das erfindungsgemäße Verfahren.

In einer bevorzugten Ausführungsform werden Pramipexol und Hilfsstoffe b) bis f) in Pulverform eingesetzt, das resultierende Gemisch ist also ein Pulvergemisch. Im Folgenden wird der Einfachheit halber jeweils Bezug genommen auf die Hilfsstoffe b) bis f), wobei entsprechend dem erfindungsgemäßen Verfahren die Hilfsstoffe d) bis f) optional sind. Der Bezug auf alle Hilfsstoffe bedeutet also nicht, dass notwendiger Weise alle diese Hilfsstoffe enthalten sein müssten.

Eine Direkttablettierung ist eine der besonders bevorzugten Ausführungsformen des erfindungsgemäßen Verfahrens. Dabei wird ein Feststoff- bzw. Pulvergemisch aus Wirkstoff und Hilfsstoffen b) bis f) zu einer Matrixtablette komprimiert bzw. verpresst. Entsprechend umfasst das erfindungsgemäße Verfahren die folgenden Schritte:
(i) Herstellen eines Gemischs umfassend Pramipexol oder ein pharmazeutisch akzeptables Salz davon a), Matrixbildner b), Tablettiermittel c), gegebenenfalls Matrixmodifikator d), gegebenenfalls Fließregulierungsmittel e) und gegebenenfalls Schmiermittel f) durch Mischen von Pramipexol oder einem pharmazeutisch akzeptables Salz davon a), Matrixbildner b), Tablettiermittel c), gegebenenfalls Matrixmodifikator d), gegebenenfalls Fließregulierungsmittel e) und gegebenenfalls Schmiermittel f), jeweils bevorzugt in fester Form und stärker bevorzugt Pulverform, und unmittelbar anschließend, d.h. ohne weitere Bearbeitung des Gemischs,
(ii) Kompression des in Schritt (i) erhaltenen Gemischs zu einer Matrixtablette.

Unter "Mischen" wird vorliegend ein Stoffvereinigungsverfahren mit dem Ziel der im Wesentlichen homogenen Verteilung verschiedener Stoffe durch Einwirkung mechanischer Kräfte verstanden. Das Mischen kann in üblichen Mischgeräten erfolgen, wie beispielsweise Wälzmischer, Schüttelmischer, Freifallmischer, Schermischer, Pflugscharmischer, Planeten-Mischkneter, Z- oder Sigma-Kneter oder Fluid- oder Intensivmischer. Bevorzugt wird ein Freifallmischer verwendet.

Die Zeit für den Schritt des Mischens (i) kann beispielsweise 0,5 Minuten bis 1 Stunde, bevorzugt 2 Minuten bis 50 Minuten, mehr bevorzugt 3 Minuten bis 30 Minuten betragen.

Der Schritt des Mischens kann beispielsweise gemeinsames Vermahlen von Pramipexol a) und Matrixbildner b) und/oder Tablettiermittel c) und/oder gegebenenfalls Matrixmodifikator d) und/oder gegebenenfalls einem oder mehreren weiteren Hilfsstoffen umfassen.

Für die Kompression (Verpressung) können die zur Tablettenherstellung üblichen Tablettiermaschinen verwendet werden. Bevorzugt werden Rundläuferpressen oder Exzenterpressen verwendet. Im Falle von Rundläuferpressen wird üblicherweise eine Presskraft von 2 bis 40 kN, bevorzugt von 2,5 bis 35 kN angewandt. Im Falle von Exzenterpressen wird üblicherweise eine Presskraft von 1 bis 20 kN, bevorzugt von 2,5 bis 10 kN, angewandt. Beispielsweise wird die Riva Piccola verwendet.

In einer bevorzugten Ausführungsform des erfindungsgemäßen Verfahrens umfasst Schritt (i) folgende Teilschritte:
(i-1) Mischen von Pramipexol oder eines pharmazeutisch akzeptablen Salzes davon a) mit Matrixbildner b), und gegebenenfalls Matrixmodifikator d),
(i-2) gegebenenfalls Granulation des in Schritt (i-1) erhaltenen Gemischs,
(i-3) Zugabe von Tablettiermittel c), gegebenenfalls Fließregulierungsmittel e) und/oder Schmiermittel f) und/oder einem oder mehreren weiteren Hilfsstoffen zu dem in Schritt (i-1) erhaltenen Gemisch oder gegebenenfalls dem in Schritt (i-2) erhaltenen Granulat.

Es ist also bevorzugt, die Inhaltsstoffe a) bis f) (und ggf. weitere Hilfsstoffe) der Tablette stufenweise miteinander zu vermischen. In einer ersten Stufe dieser Ausführungsform werden Pramipexol und Matrixbildner b) sowie gegebenenfalls Matrixmodifikator d) miteinander vermischt. Wird ein Matrixmodifikator d) verwendet, so wird er bevorzugt in dieser ersten Stufe mit Matrixbildner b) und Pramipexol a) vermischt, d.h. vor Zugabe anderer Hilfsstoffe. Bevorzugt wird kein weiterer Hilfsstoff in diesem Schritt zugegeben.

Schritt (i-1) kann auch weiter in Teilschritte unterteilt werden, indem beispielsweise zunächst Pramipexol und Matrixbildner b) miteinander vermischt werden, bevor Matrixmodifikator d) zugemischt wird, und/oder es können Teile des Matrixbildners b) stufenweise zugemischt werden.

Optional kann als Schritt (i-2) eine Granulation erfolgen. Diese Granulation kann eine Trockengranulation, eine Feuchtgranulation oder auch eine Schmelzgranulation sein, wobei Feucht- und Trockengranulation bevorzugt sind. Die beiden letztgenannten Arten der Granulation haben den Vorteil, für Wirkstoff und Hilfsstoffe schonender zu sein. Darüber hinaus ist insbesondere die Trockengranulation ein ökonomischer Prozess.

Unter "Granulieren" versteht man im Allgemeinen die Bildung gröberer oder körnigerer Haufwerke als Pulver durch Zusammenlagerung und/oder Aggregieren feinerer Pulverpartikel (Aufbaugranulation) und/oder die Bildung von feineren Granulaten durch Zerstückelung von gröberen Aggregaten (Abbaugranulation).

Die Trockengranulierung erfolgt im Allgemeinen unter Anwendung von Druck oder Temperatur. Die Feuchtgranulation erfolgt im Allgemeinen unter Verwendung von Dispersionsmitteln und optional Oberflächenstabilisatoren. Das Granulieren erfolgt im Allgemeinen in üblichen Granuliervorrichtungen, wie beispielsweise Extruder-, Lochscheiben-, Lochwalzen-, oder Wirbelschichtgranulatoren. Ebenfalls können Zwangsmischer oder Sprühtrockner verwendet werden.

Die Granulationszeit beträgt insbesondere im Fall der Nassgranulierung üblicherweise 1 Minute bis 1 Stunde, bevorzugt 2 Minuten bis 30 Minuten. Die Trockengranulierung wird üblicherweise als kontinuierlicher Prozess durchgeführt.

In einer Ausführungsform des erfindungsgemäßen Verfahrens, in welchem Schritt (i-2) eine Trockengranulation vorsieht, wird das Gemisch aus Schritt (i-1) zu einer Schülpe kompaktiert. Die Kompaktierungsbedingungen werden dabei bevorzugt so gewählt, dass die Schülpe eine Dichte von 1,03 bis 1,8 g/cm³, insbesondere von 1,05 bis 1,7 g/cm³ aufweist. Die Kompaktierung wird bevorzugt in einem Walzengranulator durchgeführt. Bevorzugt beträgt dabei die Walzkraft pro Walzenbreite 2 bis 50 kN/cm, mehr bevorzugt 4 bis 30 kN/cm, insbesondere 10 bis 25 kN/cm. Die Spaltbreite des Walzengranulators beträgt beispielsweise 0,8 bis 5 mm, bevorzugt 1 bis 4 mm, mehr bevorzugt 1,5 bis 3 mm, insbesondere 1,8 bis 2,8 mm. Anschließend wird bevorzugt die Schülpe granuliert. Die Granulierung kann allgemein mit im Stand der Technik bekannten Verfahren erfolgen.

In einer bevorzugten Ausführungsform erfolgt die Granulierung der Schülpe in einer Siebmühle. In diesem Fall beträgt die Maschenweite des Siebeinsatzes üblicherweise 0,063 bis 2 mm, bevorzugt 0,5 bis 1,5 mm, insbesondere bevorzugt 0,71 bis 1,25 mm.

Für das Trockengranulierverfahren wird bevorzugt nur das Gemisch aus Schritt (i-1) verwendet. Gegebenenfalls, aber nicht bevorzugt, können auch geringe Mengen anderer pharmazeutischer Hilfsstoffe zugesetzt werden.

Auch eine Feuchtgranulation kann mit üblichen Methoden erfolgen. Für das Feuchtgranulierverfahren wird ebenfalls bevorzugt nur das Gemisch aus Schritt (i-1) verwendet. Gegebenenfalls, aber nicht bevorzugt, können geringere Mengen weiterer pharmazeutischer Hilfsstoffe zugesetzt werden. Die Feuchtgranulation kann beispielsweise unter Verwendung eines Dispersions- bzw. Lösungsmittels der Klasse 3 erfolgen, beispielsweise Isopropanol, Ethanol, einem Gemisch aus Ethanol und Wasser, wässrigen Lösungen oder reinem Wasser. Die Verwendung reinen Wassers ist dabei bevorzugt. Sie kann beispielsweise in einem Wirbelschichtgranulator oder in einem Mischer, wie etwa einem Zwangsmischer, durchgeführt werden. Bei der Feuchtgranulation wird ebenfalls bevorzugt ein Sieb mit einer Maschenweite von 0,063 bis 2 mm, bevorzugt 0,5 bis 1,5 mm, insbesondere bevorzugt 0,71 bis 1,25 mm eingesetzt, durch das die feuchte Masse passiert wird.

Falls in Schritt (i-2) eine Feuchtgranulation durchgeführt wird, wird üblicherweise ein Schritt des "Trocknens" angewandt. Der Trockenschritt wird vorliegend als Teil des Feuchtgranulationsschritts (i-2) betrachtet.

Unter "Trocknen" versteht man im Sinne dieser Erfindung die Abtrennung von an Feststoffen anhaftenden Flüssigkeiten. Trocknen erfolgt im Allgemeinen in üblichen Trocknungsvorrichtungen, beispielsweise Schrank- oder Hordentrockner, Vakuumtrockner, Wirbelschichttrockner, Sprühtrockner oder Gefriertrockner. Bevorzugt erfolgt der Trocken- und Granuliervorgang in einem Gerät.

Bevorzugt werden die Trocknungsbedingungen so gewählt, dass der Gehalt des resultierenden Granulats an Wasser 0,1 bis 5 Gew.-% beträgt, beispielsweise 1 bis 3 Gew.-%. Bei Verwendung organischer Lösungs- bzw. Dispersionsmittel beträgt der Gehalt des resultierenden Granulats an Lösungs- bzw. Dispersionsmittel jeweils bevorzugt 1 bis 5000 ppm, beispielsweise 5 bis 100 ppm.

Die optionale Granulation in Schritt (i-2) kann auch eine Schmelzgranulation sein. Dabei kann die Schmelzgranulation beispielsweise so durchgeführt werden, dass eine Schmelze beispielsweise des Matrixbildners b) und optional des Matrixmodifikators d) und optional eines oder mehrerer weiterer Hilfsstoffe der erfindungsgemäßen Matrixtablette zu einem Wirkstoff und optional einen oder mehrere weitere Hilfsstoffe der erfindungsgemäßen Matrixtablette enthaltenden Pulver zugegeben wird, beispielsweise durch Eingießen oder Einsprühen der Schmelze.

In der eine Granulation umfassenden Ausführungsform des erfindungsgemäßen Verfahrens liegen mithin die nach dem Granulationsschritt zugegebenen Hilfsstoffe Tablettiermittel c), gegebenenfalls Fließregulierungsmittel e) und/oder Schmiermittel f) und/oder ein oder mehrere weitere Hilfsstoffe in der resultierenden Matrixtablette intergranulär vor, d.h. zwischen den Granulatkörnern.

Hierin ist die Angabe verschiedener Schritte in den erfindungsgemäßen Verfahren bevorzugt so zu verstehen, dass die Schritte in der angegebenen Reihenfolge nacheinander folgen, bevorzugt ohne Zwischenschaltung weiterer Schritte.

Die Erfindung wird durch nachstehende Beispiele unter Bezug auf die Figuren näher erläutert. Dabei zeigen
- **Figur 1**: Freisetzungsprofile der gemäß Beispiel 1 hergestellten Matrixtablette,
- **Figur 2**: Freisetzungsprofile der gemäß Beispiel 2 hergestellten Matrixtablette, und
- **Figur 3**: ein Freisetzungsprofil der gemäß Beispiel 6 hergestellten Matrixtablette.

### BEISPIELE

### Beispiel 1: Direkttablettierung

| | |
|---|---|
| Pramipexoldihydrochlorid-Monohydrat | 4,5 mg |
| Carbopol^{®} 71 G | 17,15 mg |
| Compritol^{®} 888 ATO | 146,75 mg |
| Stearinsäure | 146,75 mg |
| Calciumhydrogenphosphat | 171,35 mg |
| Magnesiumstearat | 3,5 mg |

Pramipexoldihydrochlorid-Monohydrat wurde zusammen mit Carbopol^{®}, Compritol^{®} und Stearinsäure für 10 min in einem Freifallmischer (Turbula^{®} TB 10) gemischt. Nach Zugabe von Calciumhydrogenphosphat wurde das Gemisch weitere 10 min gemischt (ggf. gesiebt) und Magnesiumstearat (ggf. über ein 0,5 mm Sieb) zugegeben. Das so erhaltene Gemisch wurde für weitere 3 min gemischt. Anschließend wurde das resultierende Gemisch auf einer Rundläuferpresse (Riva) verpresst.

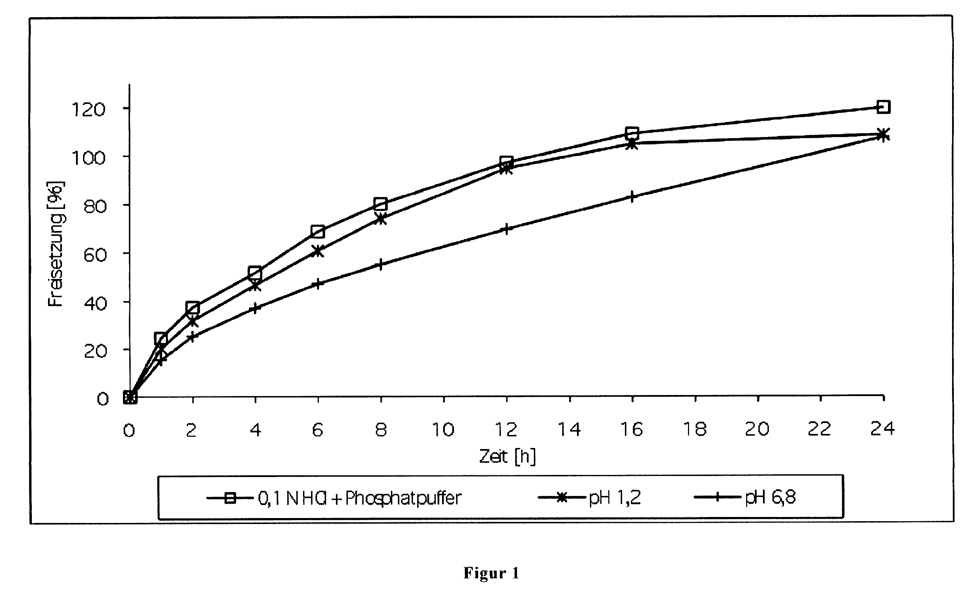

Freisetzungsprofile der so hergestellten Matrixtablette sind in Figur 1 dargestellt. Die Freisetzungsprofile wurden unter Verwendung der folgenden Messbedingungen gemessen:
1) USP Testapparatur I (Körbchen) unter Verwendung von 900 ml 0,1 N HC1 mit pH 1,2, unter Verwendung einer Drehgeschwindigkeit von 100 Umdrehungen pro Minute und bei einer Temperatur von 37°C (abgekürzt "pH 1,2");
2) USP Testapparatur I (Körbchen) unter Verwendung von 750 ml 0,1 N HC1 mit pH 1,2 für die ersten beiden Stunden und anschließendem Wechsel des pH-Werts auf pH 6,8 durch Zugabe von 250 ml Phosphatpuffer, unter Verwendung einer Drehgeschwindigkeit von 100 Umdrehungen pro Minute und bei einer Temperatur von 37°C (abgekürzt "0,1 N HC1 + Phosphatpuffer");
3) USP Testapparatur I (Körbchen) unter Verwendung von 900 ml Phosphatpuffer mit pH 6,8, unter Verwendung einer Drehgeschwindigkeit von 100 Umdrehungen pro Minute und bei einer Temperatur von 37°C (abgekürzt "pH 6,8").

### Beispiel 2: Direkttablettierung

| | |
|---|---|
| Pramipexoldihydrochlorid-Monohydrat | 4,5 mg |
| Carrageenan | 49,0 mg |
| Stearinsäure | 235,45 mg |
| Calciumhydrogenphosphat | 196,15 mg |
| Magnesiumstearat | 4,9 mg |

Pramipexoldihydrochlorid-Monohydrat wurde zusammen mit Carrageenan und Stearinsäure für 10 min in einem Freifallmischer (Turbula^{®} TB 10) gemischt. Nach Zugabe von Calciumhydrogenphosphat wurde das Gemisch weitere 10 min gemischt (ggf. gesiebt) und Magnesiumstearat (ggf. über ein 0,5 mm Sieb) zugegeben. Das so erhaltene Gemisch wurde für weitere 3 min gemischt. Anschließend wurde das resultierende Gemisch auf einer Rundläuferpresse (Riva) verpresst.

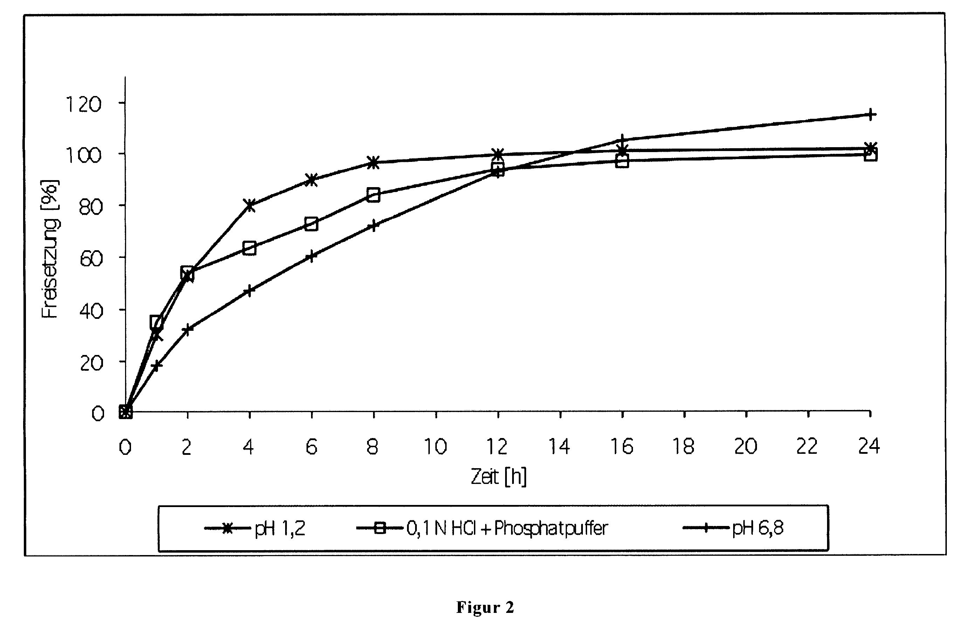

Freisetzungsprofile der so hergestellten Matrixtablette sind in Figur 2 dargestellt. Die Freisetzungsprofile wurden gemessen wie in Beispiel 1 angegeben.

### Beispiel 3: Direkttablettierung

| | |
|---|---|
| Pramipexoldihydrochlorid-Monohydrat | 4,5 mg |
| Carrageenan | 98,0 mg |
| Stearinsäure | 294,0 mg |
| Calciumhydrogenphosphat | 88,6 mg |
| Magnesiumstearat | 4,9 mg |

Pramipexoldihydrochlorid-Monohydrat wurde zusammen mit Carrageenan und Stearinsäure für 10 min in einem Freifallmischer (Turbula^{®} TB 10) gemischt. Nach Zugabe von Calciumhydrogenphosphat wurde das Gemisch weitere 10 min gemischt (ggf. gesiebt) und Magnesiumstearat (ggf. über ein 0,5 mm Sieb) zugegeben. Das so erhaltene Gemisch wurde für weitere 3 min gemischt. Anschließend wurde das resultierende Gemisch auf einer Rundläuferpresse (Riva) verpresst.

### Beispiel 4: Feuchtgranulierung

| | |
|---|---|
| Pramipexoldihydrochlorid-Monohydrat | 4,5 mg |
| Carrageenan | 45,0 mg |
| Stearinsäure | 294,0 mg |
| Calciumhydrogenphosphat | 141,6 mg |
| Magnesiumstearat | 4,9 mg |
| Wasser | 100,0 mg |

Pramipexoldihydrochlorid-Monohydrat wurde zusammen mit Carragenan und Stearinsäure für 10 min in einem Freifallmischer (Turbula^{®} TB 10) gemischt. Danach wurde Wasser zugranuliert und getrocknet. Nach Zugabe von Calciumhydrogenphosphat wurde das resultierende Granulat weitere 10 min gemischt (ggf. gesiebt) und Magnesiumstearat (ggf. über ein 0,5 mm Sieb) zugegeben. Das so erhaltene Gemisch wurde für weitere 3 min gemischt. Anschließend wurde das resultierende Gemisch auf einer Rundläuferpresse (Riva) verpresst.

### Beispiel 5: Feuchtgranulierung

| | |
|---|---|
| Pramipexoldihydrochlorid-Monohydrat | 4,5 mg |
| Carbopol^{®} 71 G | 17,15 mg |
| Compritol^{®} 888 ATO | 146,75 mg |
| Stearinsäure | 146,75 mg |
| Calciumhydrogenphosphat | 169,95 mg |
| Magnesiumstearat | 4,9 mg |
| Wasser | 100,0 mg |

Pramipexoldihydrochlorid-Monohydrat wurde zusammen mit Carbopol^{®}, Compritol^{®} und Stearinsäure für 10 min in einem Freifallmischer (Turbula^{®} TB 10) gemischt. Danach wurde Wasser zugranuliert und getrocknet. Nach Zugabe von Calciumhydrogenphosphat wurde das Granulat weitere 10 min gemischt (ggf. gesiebt) und Magnesiumstearat (ggf. über ein 0,5 mm Sieb) zugegeben. Das so erhaltene Gemisch wurde für weitere 3 min gemischt. Anschließend wurde das resultierende Gemisch auf einer Rundläuferpresse (Riva) verpresst.

### Beispiel 6: Direkttablettierung

| | |
|---|---|
| Pramipexoldihydrochlorid-Monohydrat | 4,5 mg |
| Compritol^{®} 888 ATO | 100,0 mg |
| Cutina HR^{®} | 100,0 mg |
| Cutina GMS^{®} | 100,0 mg |
| Calciumhydrogenphosphat | 180,6 mg |
| Magnesiumstearat | 4,9 mg |

Pramipexoldihydrochlorid-Monohydrat wurde zusammen mit Compritol^{®} 10 min in einem Freifallmischer (Turbula^{®} TB 10) gemischt. Nach Zugabe von Cutina HR^{®} und Cutina GMS^{®} wurde das Gemisch weitere 10 min gemischt (ggf. gesiebt). Danach wurden Calciumhydrogenphosphat und Magnesiumstearat (ggf. über ein 0,5 mm Sieb) zugegeben. Das so erhaltene Gemisch wurde für weitere 3 min gemischt. Anschließend wurde das resultierende Gemisch auf einer Rundläuferpresse (Riva) verpresst.

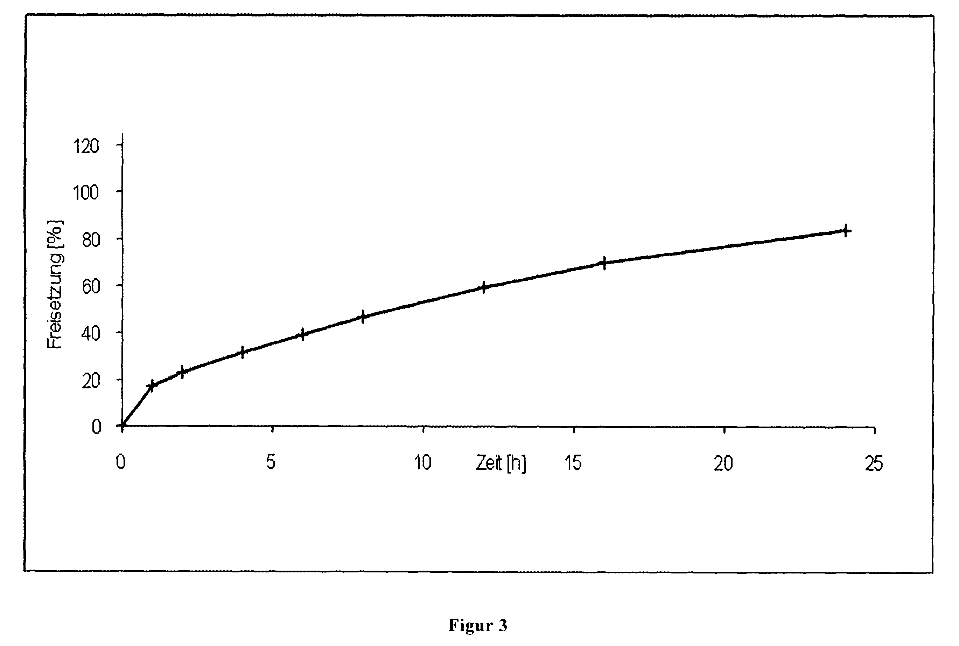

Ein Freisetzungsprofil der so hergestellten Matrixtablette in einer wässrigen Lösung mit einem pH-Wert von 6,8 ist in Figur 3 dargestellt. Das Freisetzungsprofil wurde entsprechend Beispiel 1 (Messbedingungen 3) gemessen.

### Beispiel 7: Direkttablettierung

| | |
|---|---|
| Pramipexoldihydrochlorid-Monohydrat | 4,5 mg |
| Carbopol^{®} 71 G | 17,15 mg |
| Carnaubawachs | 140,0 mg |
| Stearinsäure | 150,5 mg |
| Calciumhydrogenphosphat | 170,35 mg |
| Aerosil^{®} 200 | 3,0 mg |
| Magnesiumstearat | 4,5 mg |

Pramipexoldihydrochlorid-Monohydrat wurde zusammen mit Carbopol^{®}, Carnaubawachs und Stearinsäure für 10 min im Freifallmischer (Turbula^{®} TB 10) gemischt. Nach Zugabe von Calciumhydrogenphosphat und Aerosil^{®} wurde das Gemisch weitere 10 min gemischt (ggf. gesiebt) und Magnesiumstearat (ggf. über ein 0,5 mm Sieb) zugegeben. Das so erhaltene Gemisch wurde für weitere 3 min gemischt. Anschließend wurde das resultierende Gemisch auf einer Rundläuferpresse (Riva) verpresst.

## Patentansprüche

1. Matrixtablette zur modifizierten Freisetzung von Pramipexol, umfassend:
a) 0,05 bis 5 Gew.-%, Pramipexol oder ein pharmazeutisch akzeptables Salz davon,
b) 40 bis 80 Gew.-% eines Matrixbildners, der ausgewählt ist aus der Gruppe bestehend aus Wachsen, Fetten, Ölen, Fettsäuren mit mindestens 13 Kohlenstoffatomen, Fettalkoholen, Monoglyceriden, Diglyceriden, Triglyceriden und Gemischen davon,
c) 10 bis 50 Gew.-% Tablettiermittel,
d) 0 bis 25 Gew.-% Matrixmodifikator,
e) 0 bis 5 Gew.-% Fließregulierungsmittel, und
f) 0 bis 5 Gew.-% Schmiermittel,
bezogen auf das Gesamtgewicht der Matrixtablette.

2. Matrixtablette gemäß Anspruch 1, wobei der Matrixmodifikator d) ein pH-abhängiges Polymer umfasst.

3. Matrixtablette gemäß Anspruch 1 oder 2, wobei der Matrixmodifikator d) ausgewählt ist aus der Gruppe bestehend aus Polysacchariden, Alginaten, Polymeren der Acrylsäure und deren Derivaten, Polyacrylaten und deren Derivaten, Copolymeren aus Methacrylsäure und Methacrylsäuremethylester, Carboxymethylcellulose-Natrium, Carboxymethylcellulose-Calcium, Carboxymethylethylcellulose-Natrium, Carboxymethylethylcellulose-Calcium, Celluloseacetatphthalat, Cellulosediacetatphthalat, Cellulosetriacetatphthalat, Gummi arabicum, Xanthan Gummi, Chitinderivaten wie Chitosan, Hydroxypropylmethylcellulosephthalat, Natriumcelluloseacetatphthalat, Celluloseesterphthalat, Celluloseetherphthalat, Celluloseesteretherphthalat, Hydroxypropylcellulosephthalat, Methylcellulosephthalat, Keratin, Carrageenan und Gemischen davon.

4. Matrixtablette gemäß einem der Ansprüche 1 bis 3, wobei der Matrixmodifikator d) ausgewählt ist aus der Gruppe bestehend aus Polysacchariden und Polymeren der Acrylsäure, und bevorzugt Carrageenan ist.

5. Matrixtablette gemäß einem der Ansprüche 1 bis 4, wobei das Tablettiermittel c) Calciumhydrogenphosphat umfasst.

6. Matrixtablette gemäß einem der Ansprüche 1 bis 5, wobei der Matrixbildner b) ausgewählt ist aus der Gruppe bestehend aus Carnaubawachs, Stearinsäure, Glycerinbehenat, Glycerinmonostearat, gehärtetem Rizinusöl und Gemischen davon.

7. Matrixtablette gemäß Anspruch 6, wobei der Matrixbildner b) eine Kombination aus Glycerinbehenat, gehärtetem Rizinusöl und Glycerinmonostearat umfasst.

8. Matrixtablette gemäß einem der Ansprüche 1 bis 6, wobei der Matrixbildner b) Stearinsäure und der Matrixmodifikator d) Carrageenan ist.

9. Matrixtablette gemäß einem der Ansprüche 1 bis 6, wobei der Matrixbildner b) eine Kombination von Stearinsäure und Glycerinbehenat oder eine Kombination von Stearinsäure und Carnaubawachs ist und der Matrixmodifikator d) eine Polyacrylsäure ist.

10. Matrixtablette gemäß einem der Ansprüche 1 bis 7, wobei der Anteil an Matrixmodifikator d) 0 Gew.-% ist.

11. Matrixtablette gemäß einem der Ansprüche 1 bis 10, umfassend weniger als 1 Gew.-% Pramipexol oder ein pharmazeutisch akzeptables Salz davon.

12. Verfahren zum Herstellen einer Matrixtablette zur modifizierten Freisetzung von Pramipexol gemäß einem der Ansprüche 1 bis 11, umfassend
(i) Herstellen eines Gemischs umfassend Pramipexol oder ein pharmazeutisch akzeptables Salz davon a), Matrixbildner b), Tablettiermittel c), gegebenenfalls Matrixmodifikator d), gegebenenfalls Fließregulierungsmittel e) und gegebenenfalls Schmiermittel f),
(ii) Kompression des in Schritt (i) erhaltenen Gemischs.

13. Verfahren gemäß Anspruch 12, wobei Schritt (i) folgende Teilschritte umfasst:
(i-1) Mischen von Pramipexol oder eines pharmazeutisch akzeptablen Salzes davon a) mit Matrixbildner b), und gegebenenfalls Matrixmodifikator d),
(i-2) gegebenenfalls Granulation des in Schritt (i-1) erhaltenen Gemischs,
(i-3) Zugabe von Tablettiermittel c), gegebenenfalls Fließregulierungsmittel e) und/oder Schmiermittel f) und/oder einem oder mehreren weiteren Hilfsstoffen zu dem in Schritt (i-1) erhaltenen Gemisch oder gegebenenfalls dem in Schritt (i-2) erhaltenen Granulat.

14. Verfahren gemäß Anspruch 13, wobei in Schritt (i-2) eine Granulation erfolgt, die ausgewählt ist aus Trockengranulation, Feuchtgranulation und Schmelzgranulation.

15. Verfahren gemäß Anspruch 12, wobei das Verfahren eine Direkttablettierung darstellt, bei der in Schritt (i) ein Pulvergemisch hergestellt wird, das in Schritt (ii) direkt komprimiert wird.
